# EUROPEAN PATENT APPLICATION

(11) **EP 1 411 357 A1**
(43) Date of publication of application: **21.04.2004**
(21) Application number: 02751682.2
(22) Date of filing: 25.07.2002
(51) Int. Cl.: G01N 33/533, G01N 33/574, G01N 33/48

(54) **CANCER DIAGNOSTICS**

(30) Priority: 25.07.2001 JP 2001224054
(71) Applicant: Mitsubishi Pharma Corporation, Osaka-shi, Osaka 541-0046 (JP)
(72) Inventor: NIKI, Hisae, c/o MITSUBISHI PHARMA CORPORATION, Tokyo 103-8405 (JP); TAGAWA, Toshiaki, Mitsubishi Pharma Corporation, Tokyo 103-8405 (JP); HOSOKAWA, Saiko, c/o Mitsubishi Pharma Corporation, Tokyo 103-8405 (JP)
(74) Representative: TER MEER STEINMEISTER & PARTNER GbR
(86) International application number: PCT/JP2002/007547
(87) International publication number: WO 2003/010542

(57) **Abstract**

Provided is a diagnostic comprising a peptide or protein capable of recognizing at least a portion of a tissue section and a fluorescent substance having the below-described characteristics: i) having, in a predetermined excitation wavelength, an emission wavelength not adjacent to a wavelength region of nonspecific autofluorescence which the tissue section has; and ii) permitting simultaneous observation of the image of the peptide or protein and the image of the tissue section. A diagnostic method and diagnostic kit each of which uses the peptide or protein, and the fluorescent substance is provided. A therapeutic agent administered to a disease after selection of the appropriate agent for the disease by using the above-described method. Also, a tissue section stained by the peptide or protein and the fluorescent substance is provided. In addition, a method of analyzing expression and/or behavior of a protein by using the fluorescent substance is provided.

SEQUENCE LISTING

<110> 三菱ウェルファーマ株式会社(MITSUBISHI PHARMA CORPORATION)

<120> 癌診断薬

<130> 02030W00

<140>
<141>

<150> JP P2001-224054
<151> 2001-07-25

<160> 8

<210> 1
<211> 9
<212> PRT
<213> Homo sapiens

<400> 1
Ile Ser Ser Cys Gly Phe Tyr Trp Asn
 1               5

<210> 2
<211> 12
<212> PRT
<213> Homo sapiens

<400> 2
Ile Gly Tyr Ile Tyr Tyr Ser Gly Ser Thr Tyr Tyr

1                    5                        10

<210> 3
<211> 9
<212> PRT
<213> Homo sapiens

<400> 3
Ser Thr Arg Leu Arg Gly Ala Asp Tyr
1                    5

<210> 4
<211> 17
<212> PRT
<213> Homo sapiens

<400> 4
Lys Ser Ser Gln Ser Val Leu Tyr Asn Ser Asn Asn Lys Lys Tyr Leu
1                    5                        10                        15
Ala

<210> 5
<211> 7
<212> PRT
<213> Homo sapiens

<400> 5
Trp Ala Ser Thr Arg Glu Ser
1                    5

<210> 6

<211> 9
<212> PRT
<213> Homo sapiens

<400> 6
Gln Gln Tyr Tyr Ser Thr Pro Trp Thr
1               5

<210> 7
<211> 119
<212> PRT
<213> Homo sapiens

<400> 7
Gln Val Gln Leu Gln Glu Ser Gly Pro Gly Leu Val Lys Pro Ser Gln
1               5                   10                  15
Thr Leu Ser Leu Thr Cys Thr Val Ser Gly Gly Ser Ile Ser Ser Cys
            20                  25                  30
Gly Phe Tyr Trp Asn Trp Ile Arg Gln His Pro Gly Lys Gly Leu Glu
        35                  40                  45
Trp Ile Gly Tyr Ile Tyr Tyr Ser Gly Ser Thr Tyr Tyr Asn Pro Ser
    50                  55                  60
Leu Lys Ser Arg Val Thr Ile Ser Leu Asp Thr Ser Lys Ser Gln Phe
65                  70                  75                  80
Ser Leu Lys Leu Ser Ser Leu Thr Ala Ala Asp Thr Ala Val Tyr Tyr
                85                  90                  95
Cys Ala Arg Ser Thr Arg Leu Arg Gly Ala Asp Tyr Trp Gly Gln Gly
            100                 105                 110
Thr Met Val Thr Val Ser Ser
        115

<210> 8

<211> 114
<212> PRT
<213> Homo sapiens

<400> 8
Asp Ile Val Met Thr Gln Ser Pro Asp Ser Leu Ala Val Ser Leu Gly
1               5                   10                  15
Glu Arg Ala Thr Ile Asn Cys Lys Ser Ser Gln Ser Val Leu Tyr Asn
            20                  25                  30
Ser Asn Asn Lys Lys Tyr Leu Ala Trp Tyr Gln Gln Lys Pro Gly Gln
        35                  40                  45
Pro Pro Lys Leu Leu Ile Tyr Trp Ala Ser Thr Arg Glu Ser Gly Val
    50                  55                  60
Pro Asp Arg Phe Ser Gly Ser Gly Ser Gly Thr Asp Phe Thr Leu Thr
65                  70                  75                  80
Ile Ser Ser Leu Gln Ala Glu Asp Val Ala Val Tyr Tyr Cys Gln Gln
                85                  90                  95
Tyr Tyr Ser Thr Pro Trp Thr Phe Gly Gln Gly Thr Lys Val Glu Ile
            100                 105                 110
Lys Arg

## Description

### TECHNICAL FIELD

The present invention relates to a diagnostic, diagnostic method or diagnostic kit. In addition, the present invention pertains to a therapeutic agent for a disease, which is to be administered after selection of a medicament appropriate to the disease by using the above diagnostic, method and kit.

### BACKGROUND ART

With a view to imparting anticancer agents with higher effects and safety, researches have been pursued on cancer targeting agents using antibodies against cancer cells. It is known that corresponding to a variety of cells which become cancerous, there are many different kind of antibodies capable of recognizing them. For example, a human monoclonal antibody screened for the reactivity with gastric cancer and colorectal cancer is known as a GAH antibody (Japanese Patent Application Laid-Open No. Hei 4-346918, Japanese Patent Application Laid-Open No. Hei 5-304987).

It is presumed that upon administration of such a cancer targeting agent to a patient, higher effects and safety can be accomplished by diagnosing the reactivity with the antibody in advance by using the clinical tissue of the patient.

The reactivity of an antibody with cancer cells has conventionally been detected by a method of analyzing isolated cells through flow cytometry, or a method of analyzing a tissue section stained by the enzyme antibody method or fluorescence antibody assay without isolating cancer cells.

Flow cytometry is very useful for detecting blood-related cells or cultured cells, but the tissue section cannot be observed directly. When applied to detecting tissue sections, this method needs a cumbersome operation, and a tissue piece having a relatively large size to isolate cells from the tissue. In addition, an apparatus for flow cytometry costs high and lacks versatility.

Fluorescence antibody assay or enzyme antibody method is known as a method of analyzing a tissue section, particularly a formalin-fixed paraffin embedded section (paraffin section) which is popularly used because of convenient formation and long-term storage stability in a clinical site.

The enzyme antibody method is a method of detecting the reactivity of an antibody by reacting a section with a complex between the antibody and an enzyme such as horseradish peroxidase (HRP) and then developing an insoluble pigment derived from an enzyme substrate such as 3,3'-diaminobenzidine (DAB). In this case, however, the staining intensity depends on the conditions for the enzymatic reaction. When the reaction occurs excessively, it is sometimes difficult to judge the staining degree correctly because excessive deposition of the pigment occurs nonspecifically. It is therefore necessary to severely manage and standardize the reaction time or evaluation criteria in order to obtain highly reliable diagnostic results.

Fluorescence antibody assay, on the other hand, is a method capable of actualizing a vivid contrast and excellent from the viewpoint of quantitative determination. When a paraffin section is subjected to fluorescence observation, however, a stained antibody image cannot easily be detected because of a high autofluorescence background of the section itself. Several attempts have been made with a view to developing a fluorescence antibody assay which has the above-described features, that is, vivid contrast and excellent quantitative determination and in addition, can be used for the observation of a paraffin section.

For example, reported is a method of staining a section with a fluorescein isothiocyanate (FITC) labeled antibody, followed by counterstaining with an azo pigment to change the autofluorescence into a color utterly different from green fluorescence of FITC (Hall, C.T. Bakteriol. 184: 548-555, 1962. Hokenson, E.O. Stain Technology, 41: 9-14, 1966. Fey, H. Microbiol. 38: 271-277, 1972. Schenk, E.A. Cytochem. 22: 962-966, 1974. Hoff, H.F. Artery 6(4): 328-339, 1980). In the case of a fluorescent substance showing Stokes shift (difference (Em-Ex) between an emission wavelength (Em) available by exposing a fluorescent substance and an excitation wavelength (Ex)) longer than the excitation wavelength, to same extent, for example, R-Phycoerythrin (R-PE) or 7-amino-4-coumarin-3-acetic acid (AMCA), it is possible to separate and detect target fluorescence by installing a proper filter to a microscope, blocking fluorescence on the short wavelength side including the autofluorescence in an excitation wavelength region. Richard, et al., used a complex of Europium, one of lanthanides having a longer Stokes shift than R-PE and reduced the influence of autofluorescence of a section by the Time-Resolved Fluorescence Imaging method (Haas, R.R., J. Histochem. Cytochem. 44(10): 1091-1099, 1996).

The influence of autofluorescence can be reduced by the techniques described above, while they lead to observe only the target fluorescence as an image in a dark field. It is therefore necessary to judge it checking with information about a tissue morphology, important in diagnosis, obtained by a separate technique.

As described above, various investigations have been made to make use of the advantages of the fluorescence antibody assay for.a clinical section, but there are still some problems to be resolved from the viewpoints of convenience, quantitative determination and observation of a tissue morphology.

### DISCLOSURE OF THE INVENTION

An object of the present invention is to provide a diagnostic and method which facilitate high-sensitivity judgment using a tissue section, particularly to provide a diagnostic and method useful for selection of patients to be subjected to targeting therapy.

The present inventors have arrived at the present invention upon discovering that use of a fluorescent substance having an emission wavelength not substantially influenced by autofluorescence of a tissue section in combination with a peptide or protein enables the staining of the tissue section permitting easy judgment.

The present invention can be summarized as follows:
(1) A diagnostic, which comprises a peptide or protein capable of recognizing at least a portion of a tissue section and a fluorescent substance having the below-described characteristics i) and ii) :
   i) having, in a predetermined excitation wavelength, an emission wavelength not adjacent to a wavelength region of nonspecific autofluorescence which the tissue section has;
   ii) having fluorescence properties permitting simultaneous observation of the image of the peptide or protein and the image of the tissue section.
(2) A diagnostic as described above, wherein in a predetermined excitation wavelength, the fluorescent substance shows a Stokes shift thereof on a long wavelength side not adjacent to the wavelength region of nonspecific autofluorescence which the tissue section has.
(3) A diagnostic as described above, wherein the tissue section is a human-derived paraffin section or human-derived formalin-fixed paraffin section.
(4) A diagnostic as described above, wherein the peptide or protein is an antibody.
(5) A diagnostic as described above, wherein the antibody is a monoclonal antibody.
(6) A diagnostic as described above, wherein the monoclonal antibody is a cancer reactive monoclonal antibody.
(7) A diagnostic as described above, wherein the cancer is gastric cancer, breast cancer, colorectal cancer or esophagus cancer.
(8) A diagnostic as described above, wherein the monoclonal antibody has amino acid sequences of SEQ. ID NOS. 1, 2 and 3 of Sequence Listing in a hypervariable region of a heavy chain and amino acid sequences of SEQ. ID NOS. 4, 5 and 6 of Sequence Listing in a hypervariable region of a light chain.
(9) A diagnostic as described above, wherein the monoclonal antibody has a heavy chain variable region containing an amino acid sequence of SEQ. ID No. 7 of Sequence Listing and a light chain variable region containing an amino acid sequence of SEQ. ID No. 8 of Sequence Listing.
(10) A diagnostic as described above, wherein the monoclonal antibody is a biotin-labeled monoclonal antibody.
(11) A diagnostic as described above, wherein the Stokes shift of the fluorescent substance has a wavelength longer by at least about 80 nm than that of the tissue section.
(12) A diagnostic as described above, wherein the Stokes shift of the fluorescent substance has a wavelength longer by at least about 150 nm than that of the tissue section.
(13) A diagnostic as described above, wherein the excitation wavelength ranges from about 450 nm to about 500 nm.
(14) A diagnostic as described above, wherein the excitation wavelength ranges from about 480 nm to about 500 nm.
(15) A diagnostic as described above, wherein the excitation wavelength is about 490 nm.
(16) A diagnostic as described above, wherein the fluorescent substance is a peridinin chlorophyll protein or a fragment of the protein containing the fluorescent group portion thereof.
(17) A diagnostic as described above, wherein the peridinin chlorophyll protein is streptavidin-bound peridinin chlorophyll protein.
(18) A diagnostic as described above, which is a diagnostic for pathologic tissue.
(19) A diagnostic method, which comprises performing tissue staining with the diagnostic as described above.
(20) A diagnostic method as described above, for diagnosing the reactivity between cancer and the diagnostic.
(21) A therapeutic agent appropriate for a disease, which has been selected by using the above-described diagnostic method.
(22) A therapeutic agent as described above, wherein the disease is cancer.
(23) A therapeutic agent as described above, wherein the cancer is gastric cancer, breast cancer, colorectal cancer or esophagus cancer.
(24) An analysis method, which comprises using a fusion protein available by fusing a fluorescent substance having the below-described characteristics with a protein,
   i) having, in a predetermined excitation wavelength, an emission wavelength not adjacent to a wavelength region of nonspecific autofluorescence which the tissue section has;
   ii) having fluorescence properties permitting simultaneous observation of the image of the peptide or protein and the image of the tissue section.
(25) An analysis method as described above, wherein in a predetermined excitation wavelength, the fluorescent substance shows a Stokes shift thereof on a long wavelength side not adjacent to the wavelength region of nonspecific autofluorescence which the tissue section has.
(26) An analyzing method as described above, wherein the Stokes shift of the fluorescent substance has a wavelength longer by at least about 80 nm than that of the tissue section.
(27) An analyzing method as described above, wherein the Stokes shift of the fluorescent substance has a wavelength longer by at least about 150 nm than that of the tissue section.
(28) An analyzing method as described above, for analyzing the expression and/or behavior of the protein in a cell.
(29) A tissue section which has been subjected to tissue staining with the diagnostic as described above.
(30) A diagnostic kit, comprising the diagnostic as described above.
(31) A diagnostic kit as described above, for judging the reactivity of the therapeutic agent with cancer.
(32) A therapeutic agent for a disease, which is to be administered after selection of a medicament appropriate to the disease by using the diagnostic kit as described above.
(33) A therapeutic agent as described above, wherein the disease is cancer.
(34) A therapeutic agent as described above,
wherein the cancer is gastric cancer, breast cancer, colorectal cancer or esophagus cancer.

### BRIEF DESCRIPTION OF THE DRAWINGS

FIG. 1 is an image of gastric cancer tissue stained with a GAH antibody;
FIG. 2 is an image of gastric cancer tissue stained with a GAH antibody (method using HRP-labeled antibody);,
FIG. 3 is an image of breast cancer tissue stained with a GAH antibody;
FIG. 4 is an image of colorectal cancer tissue stained with GAH F(ab')₂;
FIG. 5 is an image of esophagus cancer tissue stained with GAH F(ab')₂;
FIG. 6 is a stained image, with a GAH antibody, of tumor grown by implantation of colorectal cancer cell line;
FIG. 7 illustrates in vivo antitumor effects for tumor grown by implantation of colorectal cancer cell line;
FIG. 8 illustrates the comparison between an image stained with FITC and that stained with PerCP;
FIG. 9 illustrates the autofluorescence spectrum analysis of a tissue section;
FIG. 10 illustrates the spectrum analysis of each of various fluorescent substances;

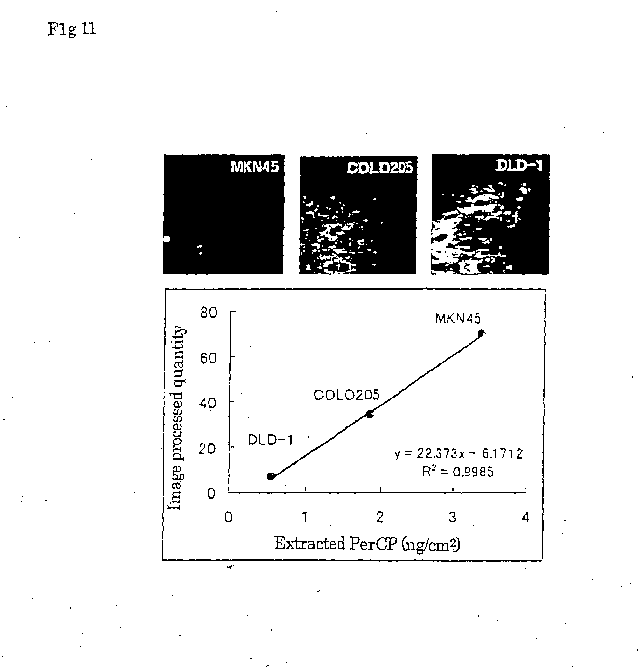

FIG. 11 illustrates the study on fluorescence quantification of an image stained with PerCP; and
FIG. 12 illustrates the comparison between an image stained with Alexa fluor 546 and that stained with PerCP.

### BEST MODE FOR CARRYING OUT THE INVENTION

The present invention will hereinafter be described specifically.

The term "tissue section" as used herein means any one of frozen section, paraffin section, formalin section, paraffin section fixed under special conditions, cultured cells or fine portions derived from tissue. Of these, preferred are human-derived paraffin section and human-derived formalin fixed paraffin section.

The term "peptide or protein capable of recognizing at least a portion of a tissue section" as used herein means, for example, an antibody, especially, a monoclonal antibody. Any monoclonal antibody is usable insofar as it is reactive with cells or tissues of cancer such as gastric cancer, breast cancer, colorectal cancer or esophagus cancer. A mouse monoclonal antibody is usable, but preferably a biotinylated human monoclonal antibody (GAH antibody) as disclosed in Japanese Patent Application Laid-Open No. Hei 5-304987.

In the GAH antibody, regions of the amino acid sequences of SEQ. ID NOS. 1, 2 and 3 of Sequence Listing are called hypervariable regions, among heavy chain variable regions, and regions of the amino acid sequences of SEQ. ID NOS. 4, 5 and 6 are called hypervariable regions among light chain variable regions. Such regions determine the specificity of immunoglobulin as an antibody and binding affinity between an antigenic determinant and antibody and they are also called "complementarity determining regions". Regions other than such hypervariable regions therefore may be derived from another antibody. In other words, an antibody having hypervariable regions similar to those of a GAH antibody can also be used in the present invention.

Accordingly, the human monoclonal antibody to be used in the present invention has amino acid sequences of SEQ. ID NOS. 1, 2 and 3 of Sequence Listing in heavy chain hypervariable regions and amino acid sequences of SEQ. ID NOS. 4, 5 and 6 of Sequence Listing in light chain hypervariable regions. These amino acid sequences are usually contained in three hypervariable regions of the heavy chain and light chain in the order of SEQ. ID NOS. 1, 2 and 3 of Sequence Listing and in the order of SEQ. ID NOS. 4, 5 and 6 of Sequence Listing, respectively from the N-terminal side. In the monoclonal antibody usable in the present invention, those modified by, for example, substitution, insertion, deletion or addition of some amino acids within a range not impairing the reactivity with cancer are also embraced.

The monoclonal antibody to be used in the present invention is available by forming hybridomas between a lymphocyte derived from a cancer patient and a mouse myeloma cell and selecting the hybridoma having the above-described specific amino acid sequences.

The hybridoma is prepared in accordance with the method of A. Imam, et al. (Cancer Research 45, 263(1985)). First, lymphocytes are isolated from a cancer-associated lymph node excised from a cancer patient and then fused with mouse myeloma cells in the presence of polyethyleneglycol. From the supernatant of the hybridomas thus obtained, those producing an antibody reactive to various cancer cell lines fixed with paraformaldehyde are selected by means of enzyme immunoassay, and cloned.

From the supernatant of the hybridomas, monoclonal antibodies are purified in the conventional manner [R.C. Duhamel, et al., J. Immunol. Methods, 31, 211(1979)] and labeled with a fluorescent substance. The reactivity with a living cancer cell line or with erythrocyte and leukocyte was detected by flow cytometry, whereby antibodies exhibiting reactivity with the living cancer cell line but not with erythrocyte and leukocyte are selected. In addition, the reactivity of antibodies with cancer cells isolated from the cancer tissue excised from a cancer patient is compared with the reactivity of the antibodies with normal cells isolated from non-cancer segment of the same tissue of the same patient, and the antibody which is bound to the cancer cells in a greater amount and does not react with normal cells or which shows reactivity as low as an antibody obtained from normal volunteer is selected.

A base sequence of a DNA encoding an antibody produced from the hybridoma selected as described above can be obtained, for example, in the following manner. In accordance with the guanidine thiocyanate-lithium chloride method [Casara, et al, DNA, 2, 329(1983)], mRNA is separated from the antibody-producing hybridoma and by using an oligo (dT) primer, its cDNA library is prepared. After (dG) tailing of the cDNA thus obtained, the antibody-encoding cDNA is amplified by the PCR method while using, as probes, poly C to be hybridized with the resulting dG tail and a consensus sequence of human antibody heavy-chain gene and light-chain gene. The terminal of the amplified DNA is made blunt. The DNA separated from an electrophoresis gel is inserted to a cloning vector such as pUC119, and the base sequence of the DNA is determined by the dideoxy method of Sanger et al [Proc. Natl. Acad. Sci. U.S.A. 74, 5463(1977)]. Based on this base sequence, the hybridoma having the above-described specific amino acid sequence can be selected.

The monoclonal antibody to be used in the present invention can also be prepared by genetic engineering technique.

The especially preferred monoclonal antibodies of the invention are those in which the heavy chain variable region and light chain variable region are represented by the amino acid sequences of SEQ. ID NOS. 7 and 8 of Sequence Listing, respectively. The base sequences encoding constant regions of the heavy and light chains are the same as those described in Nucleic Acids Research, 14, 1779(1986), The Journal of Biological Chemistry, 257, 1516(1982) and Cell, 22, 197(1980).

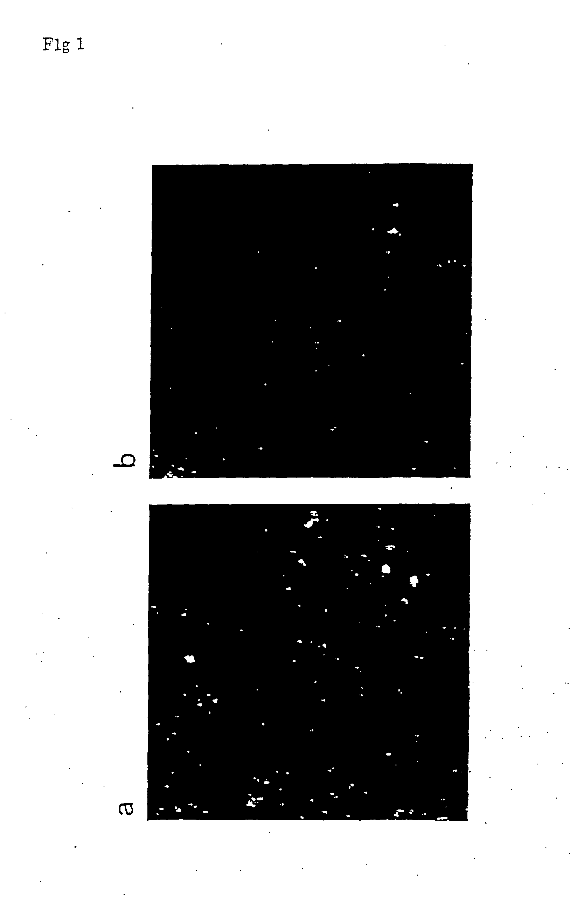

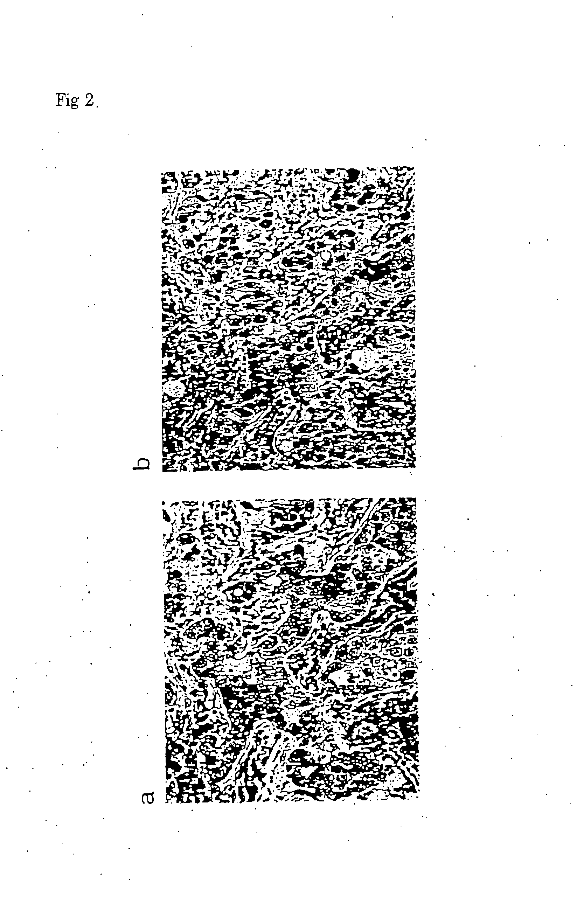

The antibody of the invention may be prepared by culturing the hybridoma producing the antibody of the invention in an eRDF, RPMI 1640 or the like medium containing fetal bovine serum. Alternatively, it may also be prepared by chemically synthesizing a gene in which DNAs encoding variable regions including the above-described specific hypervariable regions have been linked respectively with DNAs encoding the constant regions of heavy chains and light chains; inserting the gene into a known expression vector enabling the gene construction, for example as an expression vector in animal cells, pKCR (ΔE)/H and pKCRD, which can be constructed from pKCRH2 [Mishina, et al., Nature, 307, 605(1984)] in the procedure shown in FIG. 1 or FIG. 2 of Japanese Patent Application Laid-Open No. Hei 5-304987; and causing them to express in a host such as CHO cells (Chinese Hamster ovary cells). For example, a heavy chain gene having each end added with a HindIII site is inserted into the HindIII site of pKCR (ΔE)/H, while a selective marker gene such as DHFR gene is inserted into the SalI site of this plasmid. On the other hand, a light chain gene having each end added with EcoRI site is inserted into the EcoRI site of pKCRD and then the DHFR gene is also inserted into the SalI site of this plasmid. Both plasmids are introduced into cells such as CHOdhfr [Urlaub G. & Chasin L. A., Proc. Natl. Acad. Sci. U.S.A., 77, 4216(1980)] by the calcium phosphate method. The antibody can be obtained by selecting the antibody producing cells from the cells proliferated in an αMEM culture medium free of nucleotide. The antibody is purified from the cultured medium of these cells by absorbing it to a Protein A-immobilized resin such as cellulofine or agarose and then eluting it.

As the antibody, whole length antibody (whole antibody) or antibody fragment, or antibody derivative can be used. The term "antibody" as used herein embraces, as well as the whole antibody and antibody fragment (such as F(ab'), F(ab')₂ and scFv (one-strand antibody)), antibody derivatives and modified antibodies such as biotin-labeled antibody. This term must be interpreted most broadly.

In the present invention, when a peptide or protein is used as an antibody for example, no particular limitation is imposed on the kind of cancer insofar as the antibody may have reactivity therewith. Examples include gastric cancer, colorectal cancer, esophagus cancer, lung cancer, breast cancer, liver cancer, ovarian cancer, uterine cancer and pancreatic cancer. Of these, gastric cancer, breast cancer, colorectal cancer and esophagus cancer are preferred.

As the fluorescent substance to be used in the present invention, any fluorescent substance can be used insofar as it has the below-described characteristics.
i) having, in a predetermined excitation wavelength, an emission wavelength not adjacent to a wavelength region of nonspecific autofluorescence which the tissue section has;
ii) having fluorescence properties permitting simultaneous observation of the image of the peptide or protein and the image of the tissue section.

As such a fluorescent substance, use of a fluorescent substance having a Stokes shift with a longer wavelength than that of the tissue section and having a fluorescence intensity substantially enough for staining the tissue is preferred. Use of a fluorescent substance having a Stokes shift with a wavelength longer by at least about 80 nm than that of the tissue section is more preferred, with the use of a fluorescent substance having a Stokes shift with a wavelength longer by at least about 150 nm being still more preferred. The term "Stokes shift" means a difference (Em-Ex) between an emission wavelength (Em) caused by excitation of a fluorescent substance and an excitation wavelength (Ex).

In the present invention, an excitation wavelength of a fluorescent substance preferably ranges from about 450 nm to about 500 nm, more preferably from about 480 nm to about 500 nm. Within these excitation wavelength ranges, an emission wavelength ranging from about 570 nm to about 750 nm is more preferred. A fluorescent substance with an excitation wavelength of about 490 nm and an emission wavelength ranging from about 640 nm to about 750 nm is most preferred.

In the present invention, any fluorescent substance which is known or will be developed in future can be used insofar as it has the above-described excitation and emission properties. Examples include, but not limited thereto, Peridinin Chlorophyll Protein (PerCP; product of BECTON DICKINSON), Phycoerythrin R (product of Molecular Probes), Alexa Fluor 546 (product of Molecular Probes), Europium chelate Compound (product of Research Organics), red fluorescent substance available from Discosoma (product of Clontech) or variant thereof, red fluorescent substance available from Screlactinians (Riken) or variant thereof, and fluorescent substance mutant derived from *Aequorea coerulescens* (product of Clontech). Preferred fluorescent substance is PerCP of eukaryotic dinoflagellates. In the below description, sometimes PerCP is used as an example, but the fluorescent substance of the present invention is not limited thereto.

The fluorescent substance can form a chemical bond with a peptide or protein either directly or via an intermediate substance. Any method which is known or will be developed in future can be used for their binding.

Although no limitation is imposed on the conjugation method of a fluorescent substance and a peptide or protein such as antibody, the active ester method using succinimide ester, or crosslinking method using isothiocyanate, an alkylhalide, a maleimide, glutaraldehyde, periodic acid or pyridyl disulfide can be used (Eiji Ishikawa, et al., Koso Men'eki Sokuteiho, published by Igaku Shoin).

When PerCP is used as the fluorescent substance, for example, it may be bound directly to an antibody or may be bound to an antibody via an avidin-biotin complex or the like. Streptavidin-bound PerCP is more preferred.

As another embodiment of the present invention, the peptide or protein and the fluorescent substance may be bound via a water soluble polymer, for example, a polysaccharide such as dextran, agarose or cellulose or a synthetic water soluble polymer such as polyethyleneglycol.

As a further embodiment, it is possible to embed the fluorescent substance in microspheres prepared using a synthetic organic polymer compound such as polyvinylchloride, polystyrene, styrene-divinylbenzene copolymer, styrene-maleic anhydride copolymer, nylon, polyvinylalcohol, polyacrylamide, polyacrylonitrile or polypropylene, or in polymeric micelles, liposomes, lipid microspheres or the like and binding the peptide or protein to these fine particles. These fine particles may have a functional group such as amino, aminoalkyl, carboxyl, acyl or hydroxyl introduced therein.

The diagnostic of the present invention is used, for example, as a diagnostic of pathologic tissue.

In the present invention, diagnosis can be carried out by causing a diagnostic containing both the peptide or protein and the fluorescent substance to react with the tissue section to effect tissue staining and confirming the stained degree.

More specifically, the stained tissue section can be analyzed by any apparatus capable of substantially detecting a fluorescent reaction of a peptide or protein. Examples of such an apparatus include a fluorescence microscope. When both the autofluorescence and, for example, PerCP fluorescence are observed simultaneously by the fluorescence microscope, the emission wavelength of at least about 515 nm is observed at an excitation wavelength of from about 450 nm to about 500 nm. Alternatively, the autofluorescence image and red fluorescence image are observed at excitation wavelength and emission wavelength suited for them and they can be used as an overlapped image.

According to the present invention, owing to less nonspecific staining, a positive image of red fluorescence can be distinguished clearly from a stained image using human immunoglobulin which is a negative control, or the autofluorescence image (from yellowish green to deep green) of the tissue. It is therefore possible to adopt a color difference as an index for judgment.

The reactivity can be judged based on visual scoring in consideration of red fluorescence intensity and staining frequency, and also by digitalization of red fluorescence intensity by a proper image analysis computer program. It is also possible to determine the amount of fluorescence by extracting PerCP bound to the section with an acid solution. This method facilitates diagnosis of reactivity between cancer and a medicament.

The present invention also provides a therapeutic agent appropriate for a disease, which has been selected by using the above-described diagnostic method. In this case, the agent is, for example, a cancer targeting agent having an antibody. An antitumor agent as described in Japanese Patent Application Laid-Open No. Hei 5-304987 is preferred.

When the fluorescent substance of the present invention is used, fusion thereof with a protein which constitutes cells and whose expression manner or localization is unknown facilitates the pursuit of expression and/or behavior of the protein. The fusion protein may be formed in a known manner ("Idenshi Donyu & Hatsugen Kaiseki Jikkenho", published by Yodosha (1997)). This fusion protein enables examination of the expression and/or behavior of various proteins and a green fluorescent protein (GFP) popularly employed now at the same time.

In the present invention, examples of the tissue section subjected to tissue staining include pathologic tissues of cancer stained with the above-described diagnostic. Preferred examples include pathologic tissues of gastric cancer, breast cancer, colorectal cancer or esophagus cancer stained with streptavidin-bound PerCP and a GAH antibody which is a biotinylated human monoclonal antibody.

As the diagnostic kit of the present invention, those including therein the above-described diagnostic and thereby capable of judging the reactivity of a medicament with an antigen or the like can be used. The diagnostic kit will next be described with an antigen as an example.

The diagnostic kit functions based on an immunoassay of an antigen in a test sample and is used for judging the reactivity between a medicament and the antigen based on the assay value. Accordingly, the diagnostic kit of the present invention comprises at least an antibody against an antigen and a fluorescent substance, and optionally a buffer, a surfactant and a washing liquid. Examples of the immunoassay usable here include direct technique of directly detecting the reactivity of an antibody by using a fluorescence labeled antibody obtained by chemically binding a fluorescent substance to the antibody; an indirect technique of indirectly detecting the reaction of the antibody via a fluorescence-labeled secondary antibody; and avidin-biotin technique of binding an antibody and a fluorescent substance via avidin (or streptavidin)-biotin. Labeling of an antibody with a fluorescent substance may be carried out in a known manner (refer to "Zoku Seikagaku Jikken, Koza 5; Menekiseikagaku Kenkyuho", published by Tokyo Kagaku Dojin Co., Ltd.(1986), pp. 102 to 112).

The protein in the kit of the present invention may contain an antiseptic such as sodium azide, a stabilizer such as albumin, and an excipient such as saccharide. The protein can be sterilly stored at room temperature, or under a cooled or frozen state. If necessary, an inert gas such as nitrogen gas is filled upon storage. The protein is incorporated in a kit in the solution form or under a frozen or lyophilized form. If necessary, it is melted or dissolved in a solution attached to the kit upon use.

In addition to the reagents such as antibody and fluorescent substance, and buffer, surfactant, washing solution, secondary antibody, control antibody and sensitizer, materials selected as needed from the ordinarily employed ones in the assay (such as enzyme solution, substrate solution, reaction terminating solution, sample diluting solution, fading preventive, preservative for slide and the like)) may be used. A blocking agent such as serum or albumin may be incorporated in the kit in order to prevent nonspecific reaction.

The kit of the present invention can be supplemented preferably with a sterile vial of buffer, injection syringe, filter or column.

Upon fluorescence detection in the direct technique, a fluorescent-labeled antibody is added to a tissue section and after reaction and washing, the fluorescence of the section may be observed. In the indirect technique, on the other hand, the antibody is added to a tissue section and after reaction and washing, a fluorescent-labeled secondary antibody is added to visualize the antibody which has reacted with the tissue. In another embodiment, an antibody and a fluorescent-labeled secondary antibody are mixed in advance and then the resulting mixture is added to a section, whereby the reactivity can be observed. In the method using avidin-biotin, a biotinylated antibody is added to a section, and after reaction and washing, a fluorescer such as avidinated PerCP is added to visualize the antibody which has reacted with the tissue. In a further embodiment, an antibody is added to a section, and after reaction and washing, the resulting section is caused to react with a biotinylated secondary antibody, followed by the addition of a fluorescer such as avidinated PerCP to visualize the antibody which has reacted with the tissue. In a still further embodiment, a biotinylated antibody and a fluorescer such as avidinated PerCP are mixed, followed by the addition to a section, whereby reactivity can be observed. The-reactivity of the antibody can also be detected in accordance with the above-described method by using another combination, that is, a combination of avidinated antibody with a fluorescer such as biotinylated PerCP.

When the assay by the above-described method or kit has resulted in high reactivity with the tissue, it is appropriate to consider the administration of a medicament, judging that the reactivity of the tissue with the medicament may be high. Examples of the medicament include cancer targeting agent having the antibody. Preferred examples of the cancer include gastric cancer, breast cancer, colorectal cancer and esophagus cancer, and those of the cancer targeting agent include antitumor agent as described in Japanese Patent Application Laid-Open No. Hei 5-304987.

### EXAMPLES

The present invention will hereinafter be described in detail by Examples. It should however be borne in mind that the present invention is not limited to or by them provided that its essence is not exceeded.

### <Example 1: Reactivity of GAH antibody with gastric cancer tissue>

A GAH antibody as described in Japanese Patent Application Laid-Open No. Hei 5-304987 (Examples 1, 2 and 3) was labeled with a biotinylating reagent (product of Amersham Bioscience). After a paraffin section of human breast cancer tissue was de-paraffinized and then blocked by dipping in a 5%-BSA/PBS solution at room temperature for 1 hour, the resulting section was caused to react with 100 µg/ml of a biotinylated GAH antibody solution at 37°C for 2 hours. The section was washed with PBS and caused to react with 4 µg/ml of a PerCP (peridinin chlorophyll protein) labeled streptavidin solution (product of Becton/Dickinson) for 30 minutes under ice cooling in the shading. The reactivity of the GAH antibody with the breast cancer tissue section was detected as red fluorescence of PerCP having an emission wavelength of 680 nm at an excitation wavelength of 490 nm using a fluorescence microscope.

The results are shown in FIG. 1. From the gastric cancer tissue section (FIG. 1a) obtained by reacting a biotinylated GAH antibody with a PerCP labeled streptavidin solution, red fluorescence was detected. On the other hand, a gastric tissue section (FIG. 1b) obtained by reacting biotinylated human immunoglobulin as a control with a PerCP labeled streptavidin solution, no red fluorescence was detected.

### <Comparative Example 1: Reactivity of GAH antibody against gastric cancer tissue>

As a control example, a continuous section of the same tissue was de-paraffinized. After inactivation of endogenous peroxidase with aqueous hydrogen peroxide, the resulting section was dipped in a 5%-BSA/PBS solution at room temperature for 1 hour for blocking. The section was then caused to react with 10 µg/ml of each biotinylated antibody solution described in Example 1 at 37°C for 2 hours, followed by reaction with a "Vectastain elite ABC kit" (trade name; avidin-bitinyl HRP-complex of Vector Laboratories) at room temperature for 1 hour.

The reactivity of the GAH antibody with the gastric cancer tissue section was detected as a reddish brown stained image of diaminobenzidine (DAB) and the nucleus was counterstained with hematoxylin.

The results are shown in FIG. 2. From the diagram, it has been found that a reddish brown stained image was found in the GAH antibody stained image (FIG. 2a) but discrimination of it from the background was not easy and in addition, it was difficult to judge how much the reddish brown stained image was observed compared with the immunoglobulin stained image (FIG. 2b) serving as a control.

### <Example 2: Reactivity of GAH antibody against breast cancer tissue>

A GAH antibody as described in Japanese Patent Application Laid-Open No. Hei 5-304987 (Examples 1, 2 and 3) was labeled with a biotinylating reagent (product of Amersham Bioscience). A paraffin section of human breast cancer tissue was de-paraffinized and blocked by dipping it in a 5%-BSA/PBS solution at room temperature for 1 hour. The resulting section was reacted with 100 µg/ml of the biotinylated GAH antibody solution at 37°C for 2 hours. The resulting section was washed with PBS and reacted with 4 µg/ml of PerCP (peridinin chlorophyll protein) labeled streptavidin solution (product of Becton/Dickinson) for 30 minutes under ice cooling in the shading. The reactivity of the GAH antibody with the breast cancer tissue section was detected as red fluorescence of PerCP having a wavelength of 680 nm at an excitation wavelength of 490 nm using a fluorescence microscope.

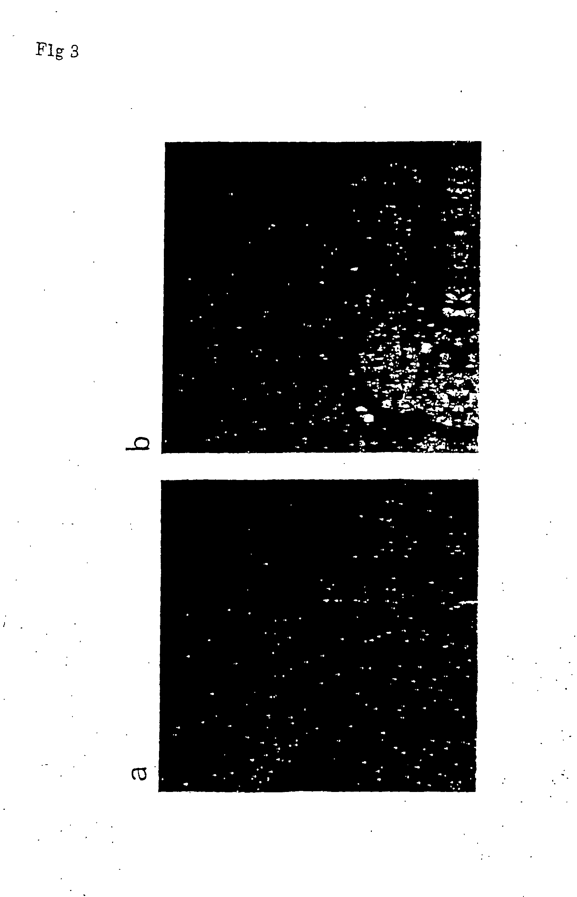

The results are shown in FIG. 3. Staining was recognized in the presence of the antibody (FIG. 3a), but not in the absence of the antibody (FIG. 3b).

### <Example 3: Reactivity of GAH F(ab')₂ with colorectal cancer tissue>

Each of GAH F(ab')₂ as described in Japanese Patent Application Laid-Open No. Hei 5-304987 (Examples 1, 2 and 3) and human IgG F(ab')₂ as a control was labeled with a biotinylating reagent (product of Amersham Bioscience). A paraffin section of human colorectal cancer tissue was de-paraffinized and blocked by dipping it in a 5%-BSA/PBS solution at room temperature for 1 hour. The resulting section was caused to react with 66 µg/ml of each of the biotinylated antibody solutions at 37°C for 2 hours. The resulting section was washed with PBS and reacted with 4 µg/ml of a PerCP-labeled streptavidin solution (product of Becton/Dickinson) for 30 minutes under ice cooling in the shading. The reactivity of the GAH antibody with the colorectal cancer tissue section was detected as red fluorescence of PerCP having a wavelength of 680 nm at an excitation wavelength of 490 nm using a fluorescence microscope.

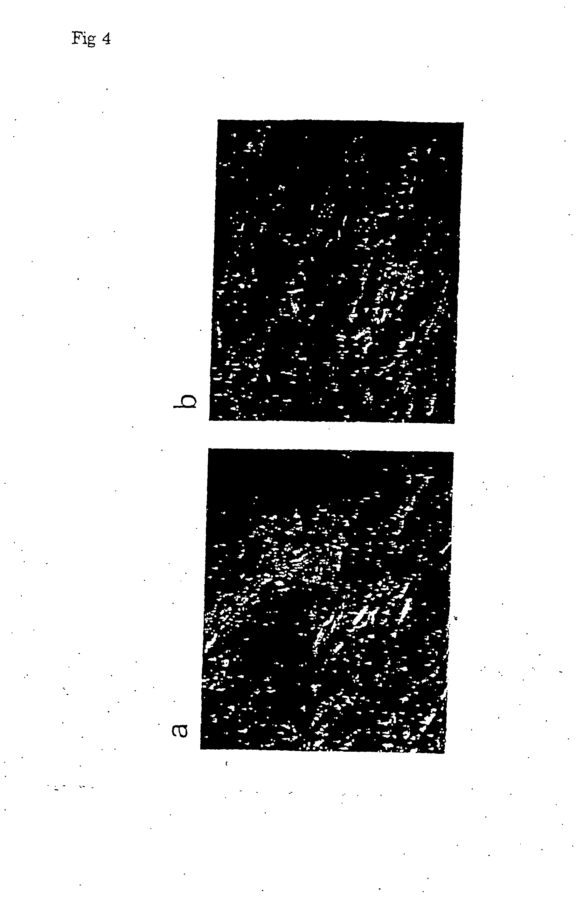

The results are shown in FIG. 4. Red fluorescence was detected from the section (FIG. 4a) of colorectal cancer tissue caused to react with the biotinylated GAH F(ab')₂ and PerCP-labeled streptavidin solution, while red fluorescence was not detected from the section (FIG. 4b) of colorectal cancer tissue caused to react with the biotinylated human IgG F(ab')₂ and PerCP-labeled streptavidin solution.

### <Example 4: Reactivity of GAH F(ab')₂ with esophagus cancer tissue>

GAH F(ab')₂ as described in Japanese Patent Application Laid-Open No. Hei 5-304987 (Examples 1, 2 and 3) was labeled with a biotinylating reagent (product of Amersham Bioscience). A paraffin section of human esophagus cancer tissue was de-paraffinized and blocked by dipping it in a 5%-BSA/PBS solution at room temperature for 1 hour. The resulting section was reacted with 66 µg/ml of a biotinylated GAH antibody solution at 37°C for 2 hours. The resulting section was washed with PBS and caused to react with a 4 µg/ml of PerCP (peridinin chlorophill protein) labeled streptavidin solution (product of Becton/Dickinson) for 30 minutes under ice cooling in the shading. The reactivity of the GAH antibody with the section of esophagus cancer tissue was detected as red fluorescence of PerCP having a wavelength of 680 nm at an excitation wavelength of 490 nm using a fluorescence microscope.

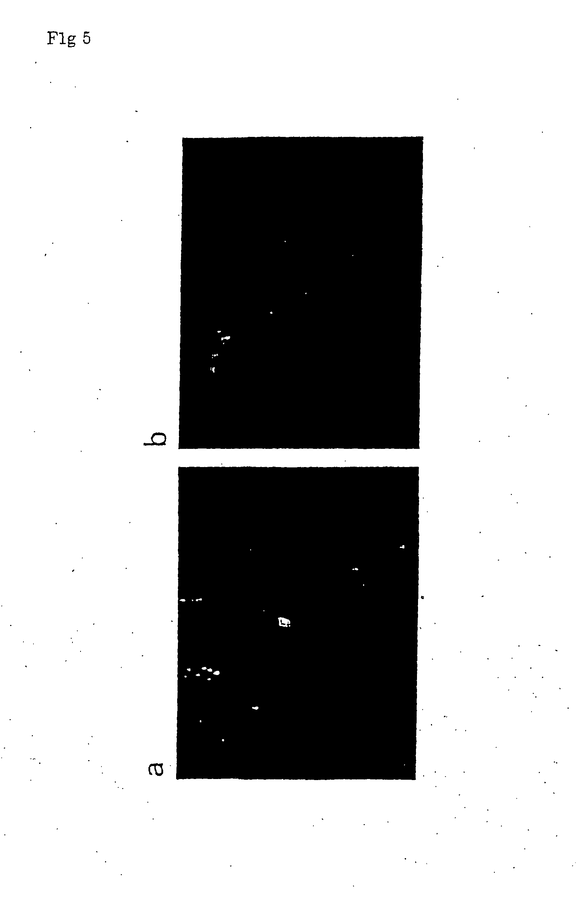

The results are shown in FIG. 5. Staining was recognized in the presence of the antibody (FIG. 5a), but not in the absence of the antibody (FIG. 5b).

### <Referential Example 1: In vivo test (observation of the tissue section) for GAH-antigen-positive or -negative colorectal cancers)>

Human colorectal cancer cell lines WiDr-Tc (GAH-antibody-reactive cell line, obtained from Institute of Development, Aging and Cancer, Tohoku University), SW837 (GAH-antibody-reactive cell line, obtained from IBL, Co., Ltd.) and Caco-2 (GAH-antibody-nonreactive cell line, product of Dainippon Pharmaceutical Co., Ltd.) were each subcutaneously implanted to a nude mouse to form tumors. A paraffin section was made using the tumor tissue. The section was de-paraffinized and blocked by dipping it in a 5%-BSA/PBS solution at room temperature for 1 hour. Then, the resulting section was caused to react with 100 µg/ml of a biotinylated GAH antibody solution at 37°C for 2 hours. The section was washed with PBS and caused to react with 4 µg/ml of a PerCP-labeled streptavidin solution (product of Becton/Dickinson) for 30 minutes under ice cooling in the shading. The reactivity of the GAH antibody was detected as red fluorescence of PerCP having a wavelength of 680 nm at an excitation wavelength of 490 nm using a fluorescence microscope.

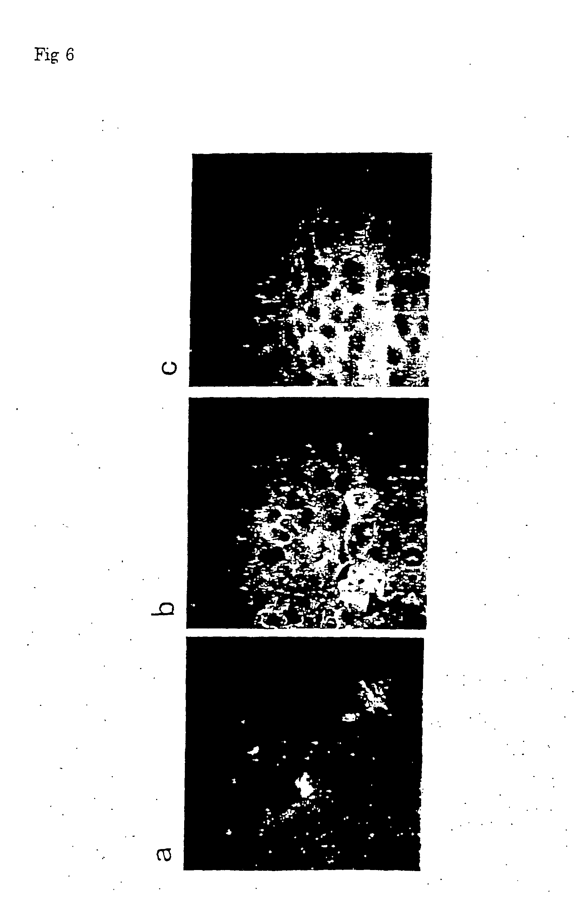

The results are shown in FIG. 6. Intense red fluorescence was detected from the cancer cells of the human colorectal cancer lines WiDr-Tc (a) and SW837(b), while red fluorescence observed from Caco-2 (c) was weaker than the former one.

### <Referential Example 2: In vivo test (antitumor effect) for GAH-antigen-positive or -negative cancers>

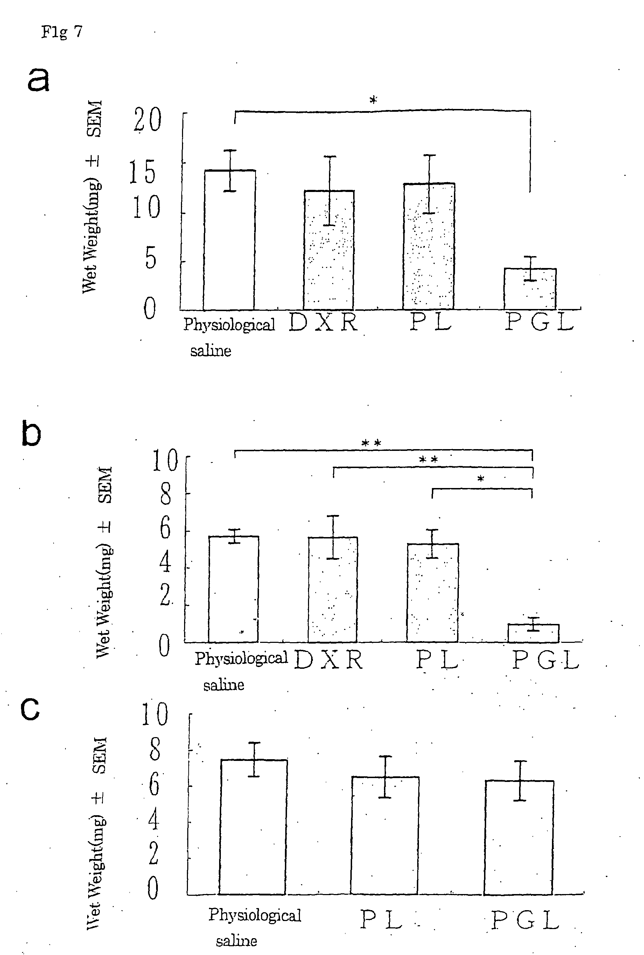

A nude mouse model was developed by implanting each of the tumor cell lines as described in Referential Example 1 under the renal capsule of the mouse and was intravenously administered with 3 mg/kg, in terms of doxorubicin (DXR), of each of doxorubicin hydrochloride (product of Kyowa Hakko Kogyo Co., Ltd.), a DXR-encapsulated PEG liposome (PL) and a DXR-encapsulated GAH-bound PEG liposome (PGL). To a control group, an equal amount of physiological saline was administered. The administration was performed once a week, three times in total. One week after the final administration, the tumor was weighed. A significant antitumor effect of PGL appeared in WiDr-Tc (FIG. 7a) and SW837 (FIG. 7b) which were GAH antibody reactive cell lines, while the effect of PGL was weak in Caco-2 (FIG. 7c) which was a GAH antibody nonreactive cell line.

The PL and PGL used in this Example were prepared in accordance with the method as described in WO99/64413 (Example 1). Described specifically, the PGL was prepared by forming a liposome having DXR encapsulated therein, and binding a thiolated GAH antibody and thiolated polyethyleneglycol (PEG) to the liposome successively. The PL was on the other hand prepared as described above except that the antibody binding operation was omitted. <Comparative Example 2>

The stained image was compared between the section of human colorectal cancer tissue using a PerCP labeled antibody and that using an FITC labeled antibody. As the antibody, biotinylated GAH F(ab')₂ was used, while biotinylated human IgG F(ab')₂ was used as a control antibody.

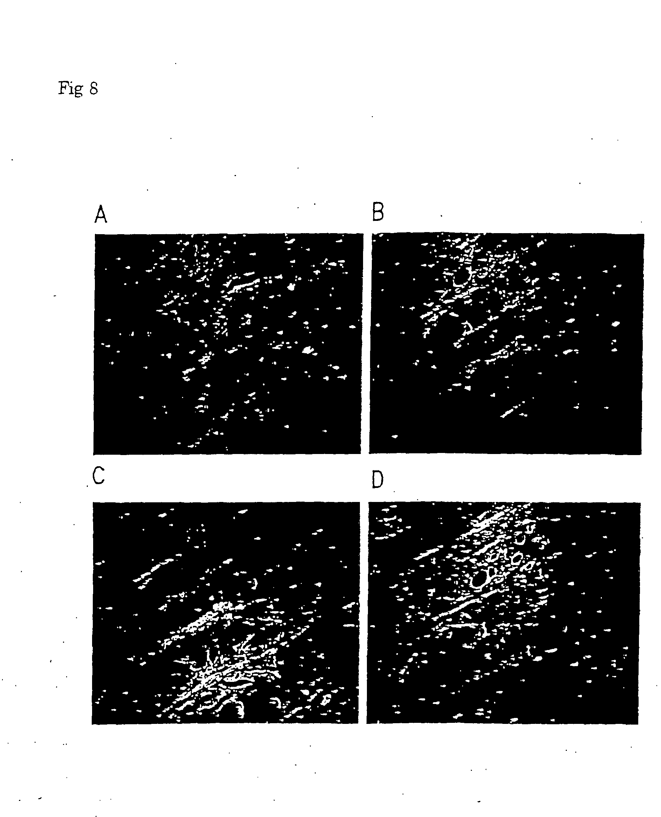

The results are shown in FIG. 8. In this diagram, indicated by A is GAH F(ab')₂ (labeled with FITC), B a control antibody (labeled with FITC), C GAH F(ab')₂ (labeled with PerCP) and D a control antibody (labeled with PerCP). The fluorescent image of the tissue section without treatment was similar to the stained image (D) of the control antibody.

### <Example 5: Autofluorescence of tissue section>

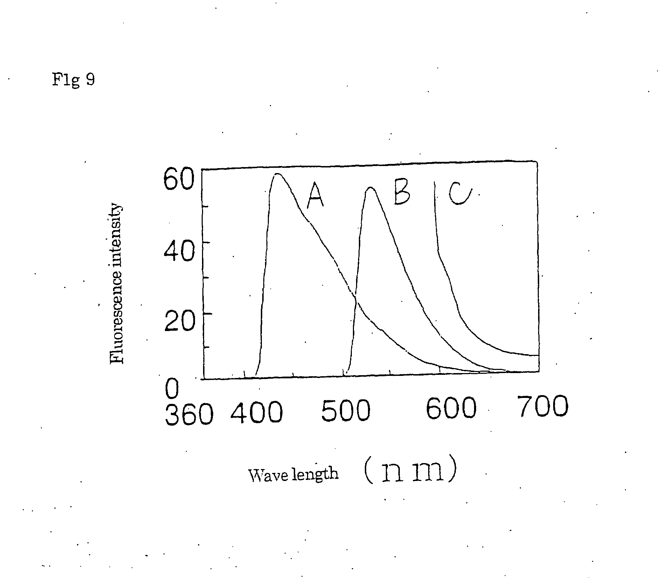

The spectrum of autofluorescence of a human colorectal cancer section prepared in a similar manner to Example 1 was measured by a spectrofluorometer (product of JASCO) attached with a solid sample holder. In FIG. 9, fluorescence spectrum at each excitation wavelength is illustrated. In this diagram, indicated at A is a fluorescence spectrum of from 360 nm to 700 nm at an excitation wavelength of 350 nm, B a fluorescence spectrum of from 500 nm to 700 nm at an excitation wavelength of 490 nm, and C a fluorescence spectrum of from 605 nm to 700 nm at an excitation wavelength of 595 nm. The Rayleigh scattering had a strong influence at each excitation wavelength, so that the photoreceptor portion of the apparatus was installed with Filter L-42 permitting transmission of fluorescence of 420 nm or greater, Filter Y-52 permitting transmission of fluorescence of 520 nm or greater, and Filter R-62 permitting transmission of fluorescence of 620 nm or greater (each, products of Kenko Co., Ltd.) for (A), (B) and (C), respectively. As illustrated in FIG. 9, it has been understood that the tissue section has strong autofluorescence within a wide range at each excitation wavelength.

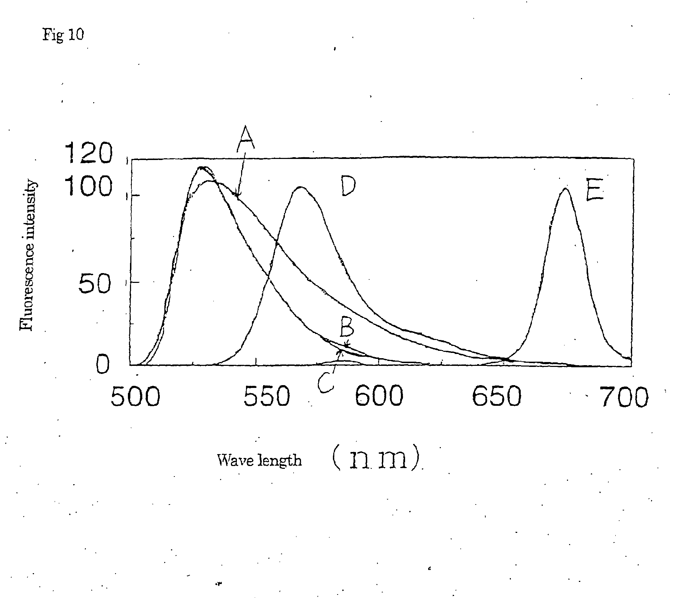

FIG. 10 illustrates comparison in the spectrum among the autofluorescence of this tissue section and various fluorochromes at an excitation wavelength of 490 nm.

In addition to the tissue section (A), the fluorescence spectrum, from 500 nm to 700 nm, of each of an FITC solution (B), an Alexa Fluor 488 solution (C), an Alexa fluor 546 solution (D) and PerCP solution (E), for each of which a solution cell was used, at an excitation wavelength of 490 nm was gained similarly. The results were shown in FIG. 10 and based on them, the spectrum was compared. The comparison has revealed that the fluorescence spectra of autofluorescence (A) and FITC (B) or Alexa Fluoro 488(C) overlapped each other. The fluorescence of Alexa Fluor 546 (D) was close to the autofluorescence. This suggests that at the above-described excitation wavelength, these three fluorescence spectra cannot clearly be separated from the autofluorescence when the emission wavelength is about 600 nm or less. On the other hand, the fluorescence spectrum (E) of PerCP was clearly separated from the autofluorescence of the tissue section, meaning that it is not influenced by autofluorescence.

### <Example 6: Extraction of fluorescent substance from tissue>

Tumors obtained by subcutaneous implantation of colorectal cancer cell lines DLD-1 (Dainippon Pharmaceutical) and COLO205 (Dainippon Pharmaceutical), and a gastric cancer cell line MKN45 (IBL Co., Ltd.) to nude mice were immunostained with a biotin labeled GAH antibody and streptavidin PerCP. From each of the tissues corresponding to 10 slides, PerCP was extracted with a 10 mM Glycine-HCl buffer, pH 1.7. Quantitative measurement of red fluorescence having an emission wavelength of 680 nm was performed by a spectrofluorometer of JASCO at an excitation wavelength of 490 nm. On the other hand, with respect to the stained image of each of the tissues, a difference between an average histogram of a red channel intensity of pixels constituting the image and an average histogram of brilliance as an index of luminance was calculated as the quantity of PerCP red fluorescence using Adobe Photoshop Ver. 5.5, showing a good correlation between the fluorescence quantitatively measured after extraction and the quantity determined by image processing.

### <Example 7: Example of anti-cytokeratin antibody>

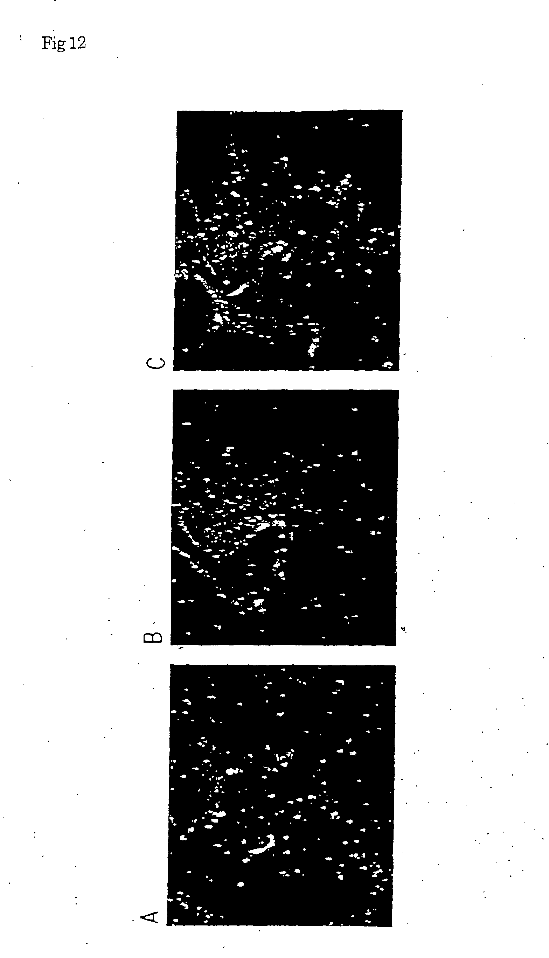

To the human gastric cancer section prepared as in Example 1, 10 µ/ml of anti-cytokeratin 8/18 mouse monoclonal antibody (product of Zymed Laboratories, Inc.) was added and they were reacted at 37°C for 1 hour. As a control, mouse IgG was used. After washing with PBS, a biotin-labeled anti-mouse IgG antibody (product of Zymed) was added, followed by reaction at room temperature for 1 hour. After washing again with PBS, the resulting section was stained with streptavidin PerCP (A) or streptavidin Alexa Fluor 546 (B,C) and fluorescence was observed as in Example 1. The stained image is shown in FIG. 12. As this diagram shows, the cancer cell in A was clearly identified by the red fluorescence of PerCP, while the cancer cell in the tissue cannot be identified clearly in B stained with Alexa Fluor 546 having yellowish red fluorescence. Staining using a control antibody resulted in the formation of a negative image similar to that of unstained tissue. As a typical example, the negative image (C) of the control antibody/Alexa Fluor 546 is illustrated.

### INDUSTRIAL APPLICABILITY

The present invention provides a diagnostic using a tissue section which diagnostic is highly sensitive and permits easy judgment. The diagnostic method using this diagnostic is very useful. It is possible to provide a therapeutic agent for a disease using the diagnostic method of the present invention for selecting a medicament appropriate to the disease; an analysis method of a protein by using a fusion protein with the fluorescent substance of the present invention; and a tissue section to be stained with the diagnostic of the present invention; a diagnostic kit for judging the affinity of a medicament with a cancer; and a therapeutic agent for a disease to be administered after selecting a medicament appropriate for the disease using the diagnostic kit.

The present application was filed, claiming priority from Japanese Patent Application 2001-224054.

## Claims

1. A diagnostic comprising a peptide or protein capable of recognizing at least a portion of a tissue section and a fluorescent substance having the below-described characteristics i) and ii):
i) having, in a predetermined excitation wavelength, an emission wavelength not adjacent, to a wavelength region of nonspecific autofluorescence which the tissue section has;
ii) having fluorescence properties permitting simultaneous observation of the image of the peptide or protein and the image of the tissue section.

2. A diagnostic of Claim 1, wherein in a predetermined excitation wavelength, the fluorescent substance shows a Stokes shift thereof on a long wavelength side not adjacent to the wavelength region of nonspecific autofluorescence which the tissue section has.

3. A diagnostic of Claim 1 or 2, wherein the tissue section is a human-derived paraffin section or human-derived formalin-fixed paraffin section.

4. A diagnostic of any one of Claims 1 to 3, wherein the peptide or protein is an antibody.

5. A diagnostic of Claim 4, wherein the antibody is a monoclonal antibody.

6. A diagnostic of Claim 5, wherein the monoclonal antibody is a cancer-reactive monoclonal antibody.

7. A diagnostic of Claim 6, wherein the cancer is gastric cancer, breast cancer, colorectal cancer or esophagus cancer.

8. A diagnostic of any one of Claims 5 to 7, wherein the monoclonal antibody has amino acid sequences of SEQ. ID NOS. 1, 2 and 3 of Sequence Listing in a hypervariable region of a heavy chain and amino acid sequences of SEQ. ID NOS. 4, 5 and 6 of Sequence Listing in a hypervariable region of a light chain.

9. A diagnostic of any one of Claims 5 to 8, wherein the monoclonal antibody has a heavy chain variable region containing an amino acid sequence of SEQ. ID No. 7 of Sequence Listing and a light chain variable region containing an amino acid sequence of SEQ. ID No. 8 of Sequence Listing.

10. A diagnostic of any one of Claims 5 to 9, wherein the monoclonal antibody is a biotin-labeled monoclonal antibody.

11. A diagnostic of any one of Claims 2 to 10, wherein the Stokes shift of the fluorescent substance has a wavelength longer by at least about 80 nm than that of the tissue section.

12. A diagnostic of Claim 11, wherein the Stokes shift of the fluorescent substance has a wavelength longer by at least about 150 nm than that of the tissue section.

13. A diagnostic of any one of Claims 1 to 12, wherein the excitation wavelength ranges from about 450 nm to about 500 nm.

14. A diagnostic of Claim 13, wherein the excitation wavelength ranges from about 480 nm to about 500 nm.

15. A diagnostic of Claim 13 or 14, wherein the excitation wavelength is about 490 nm.

16. A diagnostic of any one of Claims 1 to 15, wherein the fluorescent substance is a peridinin chlorophyll protein or a fragment of the protein containing the fluorescent group portion thereof.

17. A diagnostic of Claim 16, wherein the peridinin chlorophyll protein is streptavidin-bound peridinin chlorophyll protein.

18. A diagnostic of any one of Claims 1 to 17, which is a diagnostic for pathologic tissue.

19. A diagnostic method, which comprises performing tissue staining with the diagnostic as claimed in any one of Claims 1 to 18.

20. A diagnostic method of Claim 19, for diagnosing the reactivity between cancer and the diagnostic.

21. A therapeutic agent appropriate for a disease, which has been selected by using a diagnostic method as claimed in Claim 19 or 20.

22. A therapeutic agent of Claim 21, wherein the disease is cancer.

23. A therapeutic agent of Claim 22, wherein the cancer is gastric cancer, breast cancer, colorectal cancer or esophagus cancer.

24. An analysis method, which comprises using a fusion protein available by fusing a fluorescent substance having the below-described characteristics with a protein,
i) having, in a predetermined excitation wavelength, an emission wavelength not adjacent to a wavelength region of nonspecific autofluorescence which a tissue section has;
ii) having fluorescence properties permitting simultaneous observation of the image of the peptide or protein and the image of the tissue section.

25. An analysis method of Claim 24, wherein in a predetermined excitation wavelength, the fluorescent substance shows a Stokes shift thereof on a long wavelength side not adjacent to the wavelength region of nonspecific autofluorescence which the tissue section has.

26. An analyzing method of Claim 24 or 25, wherein the Stokes shift of the fluorescent substance has a wavelength longer by at least about 80 nm than that of the tissue section.

27. An analyzing method of any one of Claims 24 to 26, wherein the Stokes shift of the fluorescent substance has a wavelength longer by at least about 150 nm than that of the tissue section.

28. An analyzing method of any one of Claims 24 to 27, for analyzing the expression and/or behavior of the protein in a cell.

29. A tissue section which has been subjected to tissue staining with a diagnostic as claimed in any one of Claims 1 to 18.

30. A diagnostic kit, comprising a diagnostic as claimed in any one of Claims 1 to 18.

31. A diagnostic kit of Claim 30, for judging the reactivity of the therapeutic agent with cancer.

32. A therapeutic agent for a disease, which is to be administered after selection of a medicament appropriate to the disease by using the diagnostic kit as claimed in Claim 30 or 31.

33. A therapeutic agent of Claim 32, wherein the disease is cancer.

34. A therapeutic agent of Claim 33, wherein the cancer is gastric cancer, breast cancer, colorectal cancer or esophagus cancer.
